# EUROPÄISCHE PATENTSCHRIFT

(11) **EP 2 522 288 B1**
(45) Veröffentlichungstag und Bekanntmachung des Hinweises auf die Patenterteilung: **13.09.2017**
(21) Anmeldenummer: 12002634.9
(22) Anmeldetag: 16.04.2012
(51) Int. Cl.: A61B 17/88

(54) **Vorrichtung und Verfahren zum Entgasen und Austragen von Knochenzement**
Method and device for degassing and applying bone cement
Dispositif et procédé de dégazage et d'extraction de ciment osseux

(30) Priorität: 13.05.2011 DE 102011101486
(43) Veröffentlichungstag der Anmeldung: 14.11.2012
(73) Patentinhaber: Heraeus Medical GmbH, 61273 Wehrheim (DE)
(72) Erfinder: Vogt, Sebastian, Dr., 99092 Erfurt (DE); Schnieber, Tim, 60439 Frankfurt (DE)
(74) Vertreter: Heraeus IP

(56) Entgegenhaltungen:
- EP-A1- 0 397 589
- EP-A2- 1 020 167
- EP-A2- 2 210 823
- WO-A1-2009/158317
- US-A1- 2003 012 079

## Beschreibung

Die Erfindung betrifft eine Vorrichtung und ein Verfahren zum Entgasen und Austragen von Knochenzement.

Die Verwendung von PMMA-Knochenzementen geht auf grundlegenden Arbeiten von Sir Charnley zurück (Charnley, J.: Anchorage of the femoral head prosthesis of the shaft of the femur. J. Bone Joint Surg. 42 (1960) 28-30.). Der Aufbau der PMMA-Knochenzemente ist seit dem prinzipiell gleich geblieben. PMMA-Knochenzemente bestehen aus einer flüssigen Monomerkomponente und einer Pulverkomponente. Die Monomerkomponente enthält im Allgemeinen das Monomer Methylmethacrylat und einen darin gelösten Aktivator (N,N-Dimethyl-p-toluidin). Die Pulverkomponente besteht aus einem oder mehreren Polymeren, die auf Basis von Methylmethacrylat und Comonomeren, wie Styren, Methylacrylat oder ähnlichen Monomeren durch Polymerisation, vorzugsweise Suspensionspolymerisation, hergestellt sind, einem Röntgenopaker und dem Initiator Dibenzoylperoxid. Beim Vermischen der Pulverkomponente mit der Monomerkomponente entsteht durch Quellung der Polymere der Pulverkomponente im Methylmethacrylat ein plastisch verformbarer Teig. Dabei reagiert der Aktivator N,N-Dimethyl-p-Toluidin mit Dibenzoylperoxid unter Bildung von Radikalen. Die gebildeten Radikale initiieren die radikalische Polymerisation des Methylmethacrylates. Mit fortschreitender Polymerisation des Methylmethacrylates erhöht sich die Viskosität des Zementteigs, bis dieser erstarrt.

Polymethylmethacrylat-Knochenzemente können in geeigneten Mischbechern mit Hilfe von Spateln durch Vermischen des Zementpulvers mit der Monomerflüssigkeit vermischt werden. Nachteilig ist an dieser Vorgehensweise, dass Lufteinschlüsse im gebildeten Zementteig vorhanden sein können, die später eine Destabilisierung des Knochenzementes verursachen können. Aus diesem Grund wird das Vermischen von Knochenzementpulver mit der Monomerflüssigkeit in Vakuummischsystemen bevorzugt. Durch Mischen im Vakuum können Lufteinschlüsse aus dem Zementteig weitgehend entfernt werden und damit eine optimale Zementqualität erreicht werden (Breusch S.J. et al.: Der Stand der Zementiertechnik in Deutschland. Z Orthop. 1999, 137: 101-07). Im Vakuum gemischte Knochenzemente haben eine deutlich verringerte Porosität und zeigen daher verbesserte mechanische Eigenschaften. Verschiedene Vakuumzementiersysteme wurden beschrieben, von denen exemplarisch folgende genannt sind: US6033105, US5624184, US4671263, US4973168, US5100241, W99/67015, EP1020167, US5586821, EP1016452, DE3640279, WO94/26403, EP0692229, EP1005901, US558745, US5344232.

Neben der Entfernung von Luft- oder Gaseinschlüssen durch das Mischen unter Vakuum wurde zusätzlich in DE4302230C5 vorgeschlagen, den unter Vakuum gemischten Polymethylmethacrylat-Knochenzement ebenfalls unter Einwirkung von Vakuum zu sammeln. Dieses System verhindert weitgehend Lufteinschlüsse im Zement, die entstehen, wenn sich der Polymethylmethacrylat-Knochenzementteig am Kopfende der Kartuschen sammelt und die verbliebene Restluft zwischen dem Zementteig und dem Kolben am Kartuschenkopf zusammen mit dem Polymethylmethacrylat-Knochenzementteig über das Austragsrohr ausgegeben wird.

Ein anderes Konzept zur Erzielung eines weitgehend von Lufteinschlüssen freien Polymethylmethacrylat-Knochenzementes wird im gegenwärtig auf dem Markt befindlichen Vakuumzementiersystem Palamix® von der Heraeus Medical GmbH, Hanau eingesetzt. Bei diesem Zementiersystem befindet sich eine gasdurchlässige Porenscheibe im Kartuschenkopf. Durch diese Porenscheibe kann beim Austragen des Polymethylmethacrylat-Knochenzementteigs die enthaltende Luft entweichen. Dieses Konzept kann jedoch nicht bei Vakuumzementiersystemen eingesetzt werden, bei denen die Porenscheibe im Kolben integriert ist.

Neben den Polymethylmethacrylat-Knochenzementen, die auf der Basis von Pulver und Flüssigkeit hergestellt werden, sind auch Zemente bekannt, die aus Pasten hergestellt werden. Erfindungsgemäß schließt der Begriff Polymethylmethacrylat-Knochenzement sämtliche Herstellungsformen der Zemente ein, neben den aus Pulver und Flüssigkeit hergestellten insbesondere auch aus Pasten hergestellte Polymethylmethacrylat-Knochenzemente, wie sie z.B. in DE 102007050762 B3 beschrieben sind.

Die Aufgabe der Erfindung besteht somit darin, eine einfache und sichere Vorrichtung sowie ein Verfahren zum Austragen von Polymethylmethacrylat-Knochenzement bereitzustellen, die es ermöglichen, einen weitgehend von Lufteinschlüssen freien Polymethylmethacrylat-Knochenzementteig herzustellen.

Diese Aufgabe wird durch eine Vorrichtung mit den Merkmalen des Anspruchs 1 gelöst. Ferner wird diese Aufgabe durch ein Verfahren mit den Merkmalen des Anspruchs 8 gelöst. Weiterbildungen der Erfindung sind durch die Unteransprüche definiert.

Erfindungsgemäß wird eine Vorrichtung zum Entgasen und Austragen von Knochenzement bereitgestellt, deren Austragsrohr mindestens einen Bereich aufweist, der gasdurchlässig, jedoch für Knochenzement undurchlässig ist.

Bevorzugt ist dieser Bereich am hinteren Ende des Rohrs, d.h. möglichst nahe am Kartuschenkopf angeordnet.

Überraschend wurde gefunden, dass Lufteinschlüsse im Knochenzementteig bei der Bewegung entlang des gasdurchlässigen Bereichs infolge der zum Fördern des Zementteigs notwendigen Druckbeaufschlagung durch eine im oder am perforierten Austragsrohr angeordneten Porenschicht nach außen gepresst werden, während gleichzeitig der Zementteig im Inneren des Austragsrohrs in Richtung der Austragsöffnung gepresst wird. Dadurch wird am Ende des Austragsrohrs eine von Lufteinschlüssen weitgehend freien Polymethylmethacrylat-Knochenzement erhalten.

Erfindungsgemäß sind unter "Austragsrohr" Hohlkörper unterschiedlicher Form zu verstehen, die zwei einander gegenüberliegende Öffnungen aufweisen. Bevorzugt handelt es sich um einen länglichen Hohlkörper, der am vorderen und hinteren Ende, d.h. an seinen kurzen Seiten offen ist. Der Querschnitt kann quadratisch, mehreckig, oval oder rund sein. Ein Hohlkörper mit rundem oder ovalem Querschnitt ist bevorzugt. Der Körper kann sich zum Austragsende hin verjüngen.

Der gasdurchlässige Bereich wird durch ein poröses Filtermaterial gebildet. Dieses Filtermaterial muss gasdurchlässig, jedoch undurchlässig für den Zementteig sein. Das Filtermaterial ist integriert mit dem Austragsrohr ausgebildet. Möglich ist jedoch auch,
dass das Austragsrohr selbst Öffnungen aufweist, die von Filtermaterial abgedeckt sind. So kann in dem Austragsrohr zum Beispiel eine zumindest bereichsweise aus Filtermaterial bestehende Hülse angeordnet sein.

Ist das Austragsrohr konisch geformt, ist die Hülse zum Beispiel als hohler Kegelstumpf ausgebildet, so dass sie im Austragsrohr in Presspassung angeordnet ist und Öffnungen im Austragsrohr abdeckt. Dadurch, dass Austragsrohr und Hülse sich zum Austragsende hin verjüngen, sitzt die Hülse fest und kann sich nicht mit dem Polymethylmethacrylat-Knochenzementteig in Richtung der Austragsöffnung bewegen, wenn die Apparatur mit Druck beaufschlagt wird.

Es ist auch möglich, dass vom hinteren Ende des Austragsrohrs Einschnitte durch das Gewinde hindurch und bis zu einer bestimmten Höhe vorgesehen sind. Die Hülse deckt in diesem Fall die Einschnitte über die gesamte Höhe von innen ab.

Geeignete Filtermaterialien sind gesinterte poröse thermoplastische Polyolefine, wie z.B. Polyethylen und Polypropylen. Die Filter können antimikrobiell oder antiviral ausgerüstet sein. Beispiele sind in US 6551608 B2 beschrieben.

Erfindungsgemäß können Austragsrohr und Hülse jedoch auch die Form eines Hohlzylinders haben. In diesem Fall sollte die Hülse am hinteren Ende Bauelemente aufweisen, die über dem Umfang herausragen. Diese werden von hinten gegen die rückseitige Stirnfläche des Austragsrohrs gedrückt und verhindern auf diese Weise, dass sich die Hülse in Richtung Austragsöffnung bewegt. Diese Bauelemente können beliebige Formen aufweisen, sie können zum Beispiel die Form eines umlaufenden Stegs entlang des Außenrands der Hülse haben.

Die Erfindung betrifft außerdem ein Verfahren zur Herstellung eines Knochenzementteigs, der weitgehend von Gaseinschlüssen befreit ist. Dabei wird der bereits durchgemischte Knochenzementteig unter Druck entlang einer gasdurchlässigen Filterfläche transportiert. Bevorzugt erfolgt dieses Verfahren in einer oben beschrieben Vorrichtung zum Entgasen von Knochenzement.

Überraschenderweise werden Lufteinschlüsse in den Knochenzementteig bei der Bewegung entlang der gasdurchlässigen Porenschicht infolge der zum Fördern des Zementteigs notwendigen Druckbeaufschlagung durch im oder am perforierten Austragsrohr angeordneten Porenschicht nach außen gepresst, während der Zementteig im Inneren des Austragsrohr verbleibt und in Richtung des Austragsöffnung des Austragsrohrs bewegt wird. Dadurch ist es möglich, am Ende des Austragsrohrs einen von Lufteinschlüssen weitgehend freien Polymethylmethacrylat-Knochenzement zu erhalten.

Somit kann mit Hilfe der vorliegenden Erfindung auch ohne das Anlegen von Vakuum ein sehr stabiler Knochenzement hergestellt werden.

Im vorliegenden werden bevorzugte Ausführungsformen der Erfindung anhand der beiliegenden Zeichnungen genauer beschrieben. Es zeigen
- Fig. 1: eine erste erfindungsgemäße Ausführungsform im Schnitt,
- Fig. 2: eine zweite erfindungsgemäße Ausführungsform im Schnitt, und
- Fig. 3: eine dritte erfindungsgemäße Ausführungsform im Schnitt.

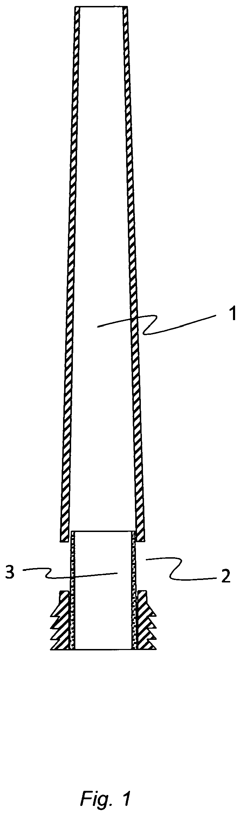

Figur 1 zeigt eine erste Ausführungsform der Erfindung mit einem Austragsrohr 1, das sich in Richtung Austragsöffnung verjüngt. Im hinteren Bereich, d.h. an dem Ende, an dem es an die Mischvorrichtung zur Herstellung des Knochenzementteigs angeschlossen wird, sind in das Austragsrohr 1 Öffnungen 2 eingelassen. Die Öffnungen 2 werden durch eine Hülse 3 abgedeckt. Die Hülse 3 besteht aus einem porösen Filtermaterial, das für Gas durchlässig, jedoch für den Knochenzement undurchlässig ist.

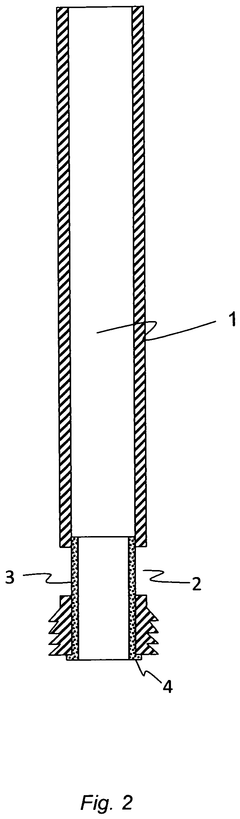

Fig. 2 zeigt eine zweite Ausführungsform der Erfindung mit einem zylindrischen Austragsrohr 1. Die Hülse 3 ist ebenfalls zylindrisch geformt und ist hinter den in das Austragsrohr eingelassenen Öffnungen 2 angeordnet. Die Hülse 3 weist an ihrem hinteren Ende einen umlaufenden vorspringenden Steg 4 auf. Der Steg 4 verhindert, dass die Hülse 3 zusammen mit dem Knochenzementteig in Richtung Austragsöffnung verschoben wird.

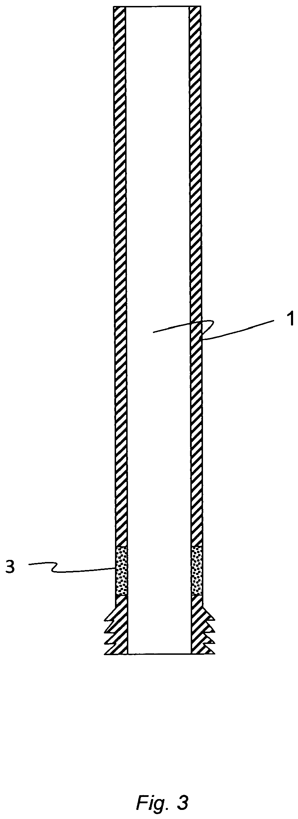

Fig. 3 zeigt eine dritte Ausführungsform, bei der der poröse Bereich 3 des Austragsrohrs integriert mit dem Austragsrohr ausgebildet ist.

## Patentansprüche

1. Vorrichtung zum Entgasen und Austragen von Knochenzement, mit einem Austragsrohr (1), wobei das Austragsrohr (1) mindestens einen Bereich aufweist, der gasdurchlässig, jedoch für Knochenzement undurchlässig ist, **dadurch gekennzeichnet, dass** der gasdurchlässige Bereich durch ein poröses Filtermaterial gebildet wird, welches integriert mit dem Austragsrohr ausgebildet ist oder Öffnungen in dem Austragsrohr abdeckt.

2. Vorrichtung nach Anspruch 1,
**dadurch gekennzeichnet, dass**
das Austragsrohr (1) Öffnungen (2) aufweist und in dem Austragsrohr eine Hülse (3) angeordnet ist, wobei Hülse zum Teil oder ganz aus gasdurchlässigem Material gefertigt ist, einen Außenumfang hat, der exakt in den Innenumfang des Austragsrohr passt und mindestens so lang ist, dass die Öffnungen (2) von innen vollständig durch die Hülse abgedeckt sind und Bereiche aus gasdurchlässigem Material hinter den Öffnungen (2) angeordnet sind.

3. Vorrichtung nach Anspruch 2,
**dadurch gekennzeichnet, dass**
das Austragsrohr (1) und die Hülse (3) konisch geformt sind.

4. Vorrichtung nach Anspruch 2 oder 3,
**dadurch gekennzeichnet, dass**
das Austragsrohr (1) und die Hülse (3) zylindrisch geformt sind.

5. Vorrichtung nach einem der Ansprüche 2 bis 4,
**dadurch gekennzeichnet, dass**
die Hülse (3) seitlich überstehende Bauelemente aufweist, die über die Öffnung des Austragsrohrs ragen.

6. Vorrichtung nach Anspruch 5,
**dadurch gekennzeichnet, dass**
die Bauelemente ein an einem Ende der Hülse nach außen vorstehender umlaufender Steg (4) sind.

7. Vorrichtung nach einem der vorhergehenden Ansprüche, **dadurch gekennzeichnet, dass**
das Filtermaterial ein gesintertes, poröses thermoplastisches Polyolefin ist.

8. Verfahren zum Entgasen und Austragen von Knochenzement in einer Vorrichtung nacht einem der Ansprüche 1 bis 7, **dadurch gekennzeichnet, dass**
der Knochenzementteig entlang der gasdurchlässigen Porenfläche in Richtung der Austragsöffnung des Austragsrohrs bewegt wird, wobei Lufteinschlüsse in dem Knochenzementteig infolge der zum Fördern des Zementteigs notwendigen Druckbeaufschlagung durch die im perforierten Austragsrohr angeordnete Porenschicht nach außen gepresst werden.

## Claims

1. Device for the degassing and dispensing of bone cement, with a dispensing tube (1), whereby the dispensing tube (1) comprises at least one region that is gas-permeable, but impermeable to bone cement, **characterised in that** the gas-permeable region is formed by a porous filter material, which is provided integrated with the dispensing tube or covers openings in the dispensing tube.

2. Device according to claim 1, **characterised in that**
the dispensing tube (1) comprises openings (2) and **in that** a sleeve (3) is arranged in the dispensing tube, whereby the sleeve is manufactured, in part or fully, from gas-permeable material, has an external circumference that fits exactly into the internal circumference of the dispensing tube, and is at least sufficiently long such that the openings (2) are fully covered from the inside by the sleeve and such that regions of gas-permeable material are arranged behind the openings (2).

3. Device according to claim 2, **characterised in that**
the dispensing tube (1) and the sleeve (3) are conical in shape.

4. Device according to claim 2 or 3, **characterised in that**
the dispensing tube (1) and the sleeve (3) are cylindrical in shape.

5. Device according to any one of the claims 2 to 4, **characterised in that**
the sleeve (3) comprises components that project on the sides and extend over the opening of the dispensing tube.

6. Device according to claim 5, **characterised in that**
the components are a circumferential fin (4) that projects outwards on one end of the sleeve.

7. Device according to any one of the preceding claims, **characterised in that**
the filter material is a sintered porous thermoplastic polyolefin.

8. Method for the degassing and dispensing of bone cement in a device according to any one of the claims 1 to 7, **characterised in that** the bone cement dough is moved along the gas-permeable pore surface in the direction of the dispensing opening of the dispensing tube, whereby air inclusions in the bone cement dough are pressed outward through the pore layer arranged in the perforated dispensing tube due to the application of pressure that is required for conveying the cement dough.

## Revendications

1. Dispositif de dégazage et d'extraction de ciment osseux, avec un tuyau d'extraction (1),
dans lequel
le tuyau d'extraction (1) présente au moins une région qui est perméable au gaz, mais imperméable pour du ciment osseux, **caractérisé en ce que** la région perméable au gaz est formée par un matériau de filtre poreux qui est réalisé de manière intégrée au tuyau d'extraction ou recouvre des ouvertures dans le tuyau d'extraction.

2. Dispositif selon la revendication 1,
**caractérisé en ce que**
le tuyau d'extraction (1) présente des ouvertures (2) et un manchon (3) est disposé dans le tuyau d'extraction, dans lequel le manchon est fabriqué en partie ou entièrement à partir de matériau perméable au gaz, a une périphérie externe qui s'adapte exactement à la périphérie interne du tuyau d'extraction et est au moins aussi long que les ouvertures (2) sont recouvertes de l'intérieur entièrement par le manchon et des régions de matériau perméable au gaz sont disposées derrière les ouvertures (2).

3. Dispositif selon la revendication 2,
**caractérisé en ce que**
le tuyau d'extraction (1) et le manchon (3) sont façonnés de manière conique.

4. Dispositif selon la revendication 2 ou 3,
**caractérisé en ce que**
le tuyau d'extraction (1) et le manchon (3) sont façonnés de manière cylindrique.

5. Dispositif selon une des revendications 2 à 4,
**caractérisé en ce que**
le manchon (3) présente des éléments de construction dépassant latéralement qui font saillie au-delà de l'ouverture du tuyau d'extraction.

6. Dispositif selon la revendication 5,
**caractérisé en ce que**
les éléments de construction sont une traverse périphérique (4) saillant vers l'extérieur à une extrémité du manchon.

7. Dispositif selon une des revendications précédentes,
**caractérisé en ce que**
le matériau de filtre est une polyoléfine thermoplastique poreuse, frittée.

8. Procédé de dégazage et d'extraction de ciment osseux dans un dispositif selon une des revendications 1 à 7,
**caractérisé en ce que**
la pâte de ciment osseux est déplacée le long de la face poreuse perméable au gaz en direction de l'ouverture d'extraction du tuyau d'extraction, dans lequel des poches d'air dans la pâte de ciment osseux sont pressées vers l'extérieur à travers la couche poreuse disposée dans le tuyau d'extraction perforé à la suite de la sollicitation en pression nécessaire pour transporter la pâte de ciment.
